Europäisches Patentamt

European Patent Office

Office européen des brevets

(11) Publication number: **0 200 197**
**B1**

(12) **EUROPEAN PATENT SPECIFICATION**

(45) Date of publication of patent specification: **22.11.90**

(21) Application number: **86105880.8**

(22) Date of filing: **29.04.86**

(51) Int. Cl.[5]: **A 61 K 7/46,** A 61 K 47/00,
C 08 J 7/02

(54) Method for impregnating a thermoplastic polymer.

(30) Priority: **02.05.85 US 729729**

(43) Date of publication of application:
**05.11.86 Bulletin 86/45**

(45) Publication of the grant of the patent:
**22.11.90 Bulletin 90/47**

(84) Designated Contracting States:
**AT BE CH DE FR GB IT LI LU NL SE**

(56) References cited:
FR-A-1 176 992
FR-A-2 361 824
US-A-4 112 151
US-A-4 492 644

CHEMICAL ABSTRACTS, vol. 84, no. 22, 31st
May 1976, page 57, abstract no. 151729c,
Columbus, Ohio, US

CHEMISTRY AND INDUSTRY, no. 12, 19th june
1982, pages 385-390, London, GB; H. BROGLE:
"CO2 as a solvent: its properties and
applications"

(73) Proprietor: **HERCULES INCORPORATED**
**Hercules Plaza**
**Wilmington Delaware 19894 (US)**

(72) Inventor: **Sand, Michael Leonard**
**1300 N. Harrison Street, Apt. A400**
**Wilmington Delaware 19806 (US)**

(74) Representative: **Lederer, Franz, Dr. et al**
**Van der Werth, Lederer & Riederer**
**Patentanwälte Lucile-Grahn-Strasse 22**
**D-8000 München 80 (DE)**

**Description**

This invention relates to a method for impregnating a thermoplastic polymer with an impregnation material such as a fragrance or pest control agent or pharmaceutical composition.

Thermoplastic polymers that are impregnated with a desirable fragrance such as a perfume are well known. The major difficulty with making such thermoplastic polymers lies in impregnating the polymer with sufficient fragrance material without adversely affecting either the polymer or the fragrance material.

With some polymers impregnating can be achieved by immersing the polymer in the fragrance material or a solution containing the fragrance material under pressure. However, many polymers, notably thermoplastic polymers, are difficult to impregnate in this manner, even at elevated temperatures. For instance, exposure of polypropylene to fragrant alcohols, esters or aldehydes for 2 hours under a nitrogen pressure of over 17200 kPa introduces only 0.2% by weight of the fragrance material.

For example, U.S. Patent No. 3,926,655 discloses a perfumed polyamide resin that is made by heating the polyamide resin until it is pourable and stirrable then blending a perfume oil therein. Similarly, U.S. Patent No. 4,095,031 discloses perfumed polymers of ethylene and polar monomer made by heating the polymer until it is free-flowing and then blending a perfumed oil therein.

However, when an elevated temperature is required, the choice of a fragrance material is limited to those that are not heat-sensitive. Thus when thermoplastic polymers are impregnated by introducing the fragrance material into a polymer melt prior to compression or injection molding, the high temperatures of the polymer melt can volatilize, degrade or otherwise adversely affect the fragrance material.

A need also exists for impregnating other materials, such as pharmaceuticals and pest control agents, into polymers for controlled release. For example, Langer, in Chem. Eng. Commun., Vol. 6, pages 1—48 (1980), discloses the application of polymeric delivery systems in pharmaceutical, agricultural and fragrance uses. Langer, et al., in Biomaterials 2, October, 201—214 (1981) discloses some medical control release applications.

Grubb, U.S. Patent 3,725, 311; Wilbert, et al., U.S. Patent 3,567,119; and Engel, U.S. Patent 3,688,985 each discloses impregnated polymer systems.

The use of carbon dioxide to extract or dissolve fragrances and pharmaceutical materials is known. For instance, Peyron, in Parfums, Cosmetiques, Aromes, No. 55, 47—54, February-March (1984), discusses extraction of fragrance and pharmaceutical materials from natural products using carbon dioxide. Sims, in U.S. Patent 4,281,171 (July 28, 1981) discloses that pyrethrins, which are useful as pest control agents, can be extracted from pyrethrum flowers with liquid carbon dioxide. Stahl, in Rev. Latinoamer. Quim. *11*, 1—7 (1980), discloses that some alkaloids dissolve at 40°C in carbon dioxide or nitrous oxide used in its supercritical state, that is, under conditions above its critical temperature and at a pressure above its critical pressure. (The critical temperature ($T_c$) is the temperature above which the gas cannot be liquified at any pressure. The critical pressure ($P_c$) is the pressure required to condense the gas at $T_c$).

U.S. Patent Patent No. 4,167,589 discloses another process using carbon dioxide for extraction of materials from natural products, that also includes reimpregnation of aromatics. The process comprises first extracting the aromatic content from tea with a solvent, preferably carbon dioxide, in its supercritical state, separating the aromatic content from the solvent by reducing the pressure and then extracting caffeine from the tea residue with the moist solvent in its supercritical state. The aromatic content is then redissolved in the solvent in its supercritical gaseous state which is then liquified at a subcritical temperature and brought into contact with the tea. The tea residue is reimpregnated with its aromatic content by drawing off the subcritical solvent from the liquid phase containing the tea residue, and the tea is freed from the solvent by slow evaporation.

An entirely different problem is encountered when the impregnation material is to be absorbed by a solid material that is normally resistant to such absorption, such as a thermoplastic polymer.

According to the invention, a method for impregnating a solid with an impregnation material by contacting the solid with a volatile solvent in which the impregnation material is dissolved and then the pressure is reduced, is characterized in that the solid is a thermoplastic polymer and the solvent is also a swelling agent for the polymer, the polymer is contacted with and swollen by the solvent containing the impregnation material in solution at or near the supercritical conditions of temperature and pressure for the solvent, and the pressure is reduced to diffuse the solvent out of the swollen thermoplastic polymer, the impregnation material being a fragrance or pest control agent or pharmaceutical agent and being less volatile than the volatile solvent.

The expression "at or near supercritical conditions of temperature and pressure" is generally familiar to those skilled in the art, and specifically means either 1) at a temperature between its critical temperature and about 20°C below its critical temperature and a pressure at or above the vapor pressure of the solvent in its liquid state at that temperature, or 2) approximately at its critical temperature and pressure, or 3) under conditions of temperature and pressure at which the solvent is in its supercritical gaseous state, namely, at a temperature between its critical temperature and about 100°C, and a pressure between its critical pressure an about 68950 kPa.

Preferably, the temperature of the solvent is in the range from about 40°C to about 60°C, which ensures a convenient rate of swelling of the polymer. The preferred pressure is in the range from about 6900 kPa to about 34500 kPa. The choice of the operating temperature within the defined limits depends on such factors as the need to avoid a temperature that will degrade or otherwise adversely affect the impregnation material or the thermoplastic polymer.

The choice of the operating pressure within the defined limits also depends on practical considerations. For instance, at lower pressures the rate of swelling is lower, while at higher pressures the cost of operating equipment increases. The defined upper limit of pressure is dictated by economic considerations.

A volatile solvent that can "swell" a thermoplastic polymer at or near the supercritical conditions for the solvent is generally understood to mean one that dissolves in the polymer; in other words, it increases the diffusivity of the solvent within the polymer, possibly by separating the crystalline polymeric chains. It normally chauses a measurable change in the glass transition temperature of the polymer.

Preferably the volatile solvent/swelling agent is lipophilic, and the impregnation material is fat soluble. The preferred solvent /swelling agents are carbon dioxide (vapor pressure of 1 atm [101.32 kPa] at −78.4°C, $P_c$ = 1071 psi, [7384 kPa], $T_c$ = 31.0°C); ethylene (B.P. = −103.7°C, $P_c$ = 742 psi, [5116 kPa], $T_c$ = 9.9°C.); and nitrous oxide (B.P. = −88.5°C, $P_c$ 1052 psi, [7253 kPa] $T_c$ = 36.4°C). Carbon dioxide is the most preferred solvent because of its unusually high vapor pressure, its nonflammability and its low cost. Furthermore, because carbon dioxide is physiologically an environmentally safe and has no odor, any small amounts left in the thermoplastic polymer have no adverse effects.

If the solvent is carbon dioxide, the extreme ranges of operating temperatures and pressures may be respectively from about 11.6°C to 100°C, and from about 3450 kPa (its vapor pressure at 11.6°C) to about 68950 kPa.

Nitrous oxide is also a preferred solvent/swelling agent for impregnation materials that are uniquely compatible with it, as will be apparent to those skilled in the art. In general the choice of a solvent/swelling agent will depend on parameters similarly apparent, such as its compatibility with the impregnation material and the thermoplastic polymer, its volatility, and its ability to swell the thermoplastic polymer.

The fragrance, pest control agent or pharmaceutical impregnation material is dissolved by contacting it with the volatile solvent/swelling agent at or near the supercritical conditions. The preferred solution conditions are similar to those suitable for swelling the polymer.

Fragrances suitable for the present invention include the conventional fragrance materials that can be dissolved in the volatile solvent/swelling agent under the appropriate conditions. These are well-known complex mixtures of compounds that may include esters, ethers, aldehydes, alcohols, unsaturated hydrocarbons, and terpenes. Examples of suitable fragrance are musk, rose oil, honeysuckle oil, pine oil, and jasmine or oak musk. Examples of the other kinds of impregnation materials are pheromones and pyrethrins as pest control agents and scopolamine as a pharmaceutical composition.

Typical solubilities of individual fragrance, pest control agent and pharmaceutical compounds are well known to those skilled in the art. Solubilities in solvents at or near the supercritical conditions are generally known to be dependent on temperature and pressure.

The amount of fragrance, pest control agent or pharmaceutical impregrantion material in the impregnated thermoplastic polymer can be controlled; the greater the concentration of impregnation material in the volatile solvent/swelling agent, the greater the concentration of impregration material in the final thermoplastic polymer.

It is apparent that the concentration cannot be so great that the behavior of the volatile solvent/swelling agent at or near supercritical conditions is adversely affected. Preferably, the concentration of impregnation material in the volatile solvent/swelling agent is from about 0.1% to about 50% by weight, most preferably about 10% to about 30% by weight for fragrance impregnation materials, and from about 0.1% to about 3% by weight for pharmaceutical materials. The optimal concentration is a matter of choice by one skilled in the art depending on the intended use of the impregnated polymer.

Any thermoplastic polymer swellable by a suitable volatile solvent for the impregnation material can be employed, such as polypropylene, polyethylene, ethylene-vinyl acetate (EVA) copolymers and ethylene-ethyl acrylate copolymers. The thermoplastic polymer is swollen by contacting it at or near the supercritical conditions with the solvent/swellling agent containing the impregnation material, which results in diffusion of the solvent/swelling agent into the polymer. The efficiency of the swelling is increased with increasing temperature at a constant pressure.

Another factor having bearing on the impregnation is the size and form of the polymeric matrix. A wise variety of solid forms, ranging from polymer flakes and pellets to the molded articles themselves. However, since the time required for the transfer of impregnation material is proportional to the ratio of external surface area to volume, there is a practical limit on the size of the molded article.

The polymer is impregnated in an autoclave or other highpressure vessel. After contacting the polymer, the volatile solvent/swelling agent is separated from the polymer leaving the impregnation material behind. Because of the volatility of the solvent/swelling agents employed, separation is easily accomplished merely by lowering the pressure.

If an autoclave containing perfumed molded products is vented rapidly, the physical appearance of the product can be adversely affected. The extent to which that occurs depends on the type of polymer and other factors including pressure, temperature, dimensions of the polymer product, rate of venting, and amounts of impregnation material and supercritical fluid dissolved in the polymer.

Adjustment of the above processing parameters will determine other characteristics of the final product. For instance, microvoids can be created in molded polymer pieces by controlled rapid venting of the swelling agent. The microvoids may act as reservoirs for impregnation material.

Such microvoids tend not to form at the surface of the polymer article, thus retaining an outer shell that can resist diffusion from internal reservoirs. Such a structure will tend to release the impregnation material more uniformly over time than a uniform polymer matrix would.

The following examples illustrate the invention, which, however is not limited to these specific examples.

Example 1

Polymeric plaques having the composition shown in Table 1 and a surface to volume ratio of 10 cm$^{-1}$ are weighed and placed into a cylindrical, mesh basket. The basket is attached to the agitator shaft of a stirred autocalve. A tank containing compressed $CO_2$ is attached to a feed line and the autoclave is pumped to an intermediate pressure. The compressed gas feed line is then directed through a small vessel that contains a typical fragrance material composed of esters, alcohols, aldehydes and fixatives and the $CO_2$ becomes laden with the fragrance material, such as fragrant alcohols (for example, linalool), esters (for example, linalyl acetate), and aldehydes (for example, anisaldehyde) and blends thereof. The fragrance material-laden $CO_2$ is forced into the autoclave.

The results shown in Table I for fragrance under $N_2$ pressure are given as the amount of fragrance in the polymer product as a weight percent increase of polymer from starting polymer to swollen polymer when impregnated through immersion in fragrance under $N_2$ pressure. Results for the fragrance/$CO_2$ mixture are given as the amount of fragrance in the polymer product in the absence of any significant amount of residual $CO_2$ as a weight percent increase of polymer from starting polymer to swollen polymer when swollen under 92% carbon dioxide and 8% fragrance.

Table I shows impregnation of fragrance (by weight percent increase) into compression molded polymer plaques with $N_2$ or $CO_2$ at 25°C., 2500 psi [17 237 kPa] for 2 hours for copolymers I—III. The weight ratios of $CO_2$ to fragrance to polymer is 60:5:1.

TABLE I

| Polymer | Impregnation Medium: | |
| | Fragrance under $N_2$ pressure | Fragrance/$CO_2$ mixture* |
| --- | --- | --- |
| Copolymer I [ethylene-vinyl acetate (9% vinyl acetate)] | 0.74% | 0.89% |
| Copolymer II [ethylene-vinyl acetate (18% vinyl acetate)] | 4.37% | 6.49% |
| Copolymer III [ethylene-ethyl acrylate (18% ethyl acrylate)] | 3.80% | 4.86% |

*8 weight percent fragrance.

Example 2

Example 2 is carried out under the same conditions as Example 1 except that the contacting is carried out at 60°C. The weight ratio of $CO_2$: fragrance:polymer is 50:5:1. The composition of the polymer plaques and the percentage weight increase are shown in Table II.

Table II shows the results of impregrantion of fragrance (by weight percent increase) into compression molded polymer plaques with $N_2$ or $CO_2$ at 60°C and 2500 psi [17 237 kPa] for 2 hours.

TABLE II

| Polymer | Impregnation Medium: | |
| | Fragrance under $N_2$ pressure | Fragrance/$CO_2$ mixture* |
| --- | --- | --- |
| Copolymer I [ethylene-vinyl acetate (9% vinyl acetate)] | 4.19% | 8.65% |
| Polypropylene | 0.20% | 1.42% |
| Polyethylene | 1.35% | 2.65% |

*9 weight percent fragrance

Table III shows the polymer characterizations of the polymers used in Examples 1 and 2 as referred to in Tables I and II.

TABLE III

The melt flow rate ($I_2$ at 230°C) and the melt index ($I_2$ at 190°C) in decigrams per minute (dg/minute) is determined by ASTM test method D 1238.

1) Polypropylene — melt flow rate ($I_2$ at 230°C) is 3.3 (dg/minute), density = 0.903 g/cc.

2) Polyethylene — melt index ($I_2$ at 190°C) is 7.0 (dg/minute), density = 0.917 g/cc.

3) Copolymer I — melt index ($I_2$ at 190°C) is 3.0 (dg/minute), density = 0.930 g/cc [Ethylene-vinyl acetate (9% vinyl acetate)].

4) Copolymer II — melt index ($I_2$ at 190°C) is 150 (dg/minute), density = 0.937 g/cc [Ethylene-vinyl acetate (18% vinyl acetate)].

5) Copolymer III — melt index ($I_2$ at 190°C) is 20.0 (dg/minute), density = 0.931 g/cc [Ethylene-ethyl acrylate (18% ethyl acrylate)].

## Claims

1. A method for impregnating a solid with an impregnation material by contacting the solid with a volatile solvent in which the impregnation material is dissolved and then the pressure is reduced, characterized in that the solid is a thermoplastic polymer and the solvent is also a swelling agent for the polymer, the polymer is contacted with and swollen by the solvent containing the impregnation material in solution at or near the supercritical conditions of temperature and pressure of the solvent, and the pressure is reduced to diffuse the solvent out of the swollen thermoplastic polymer, the impregnation material being a fragrance or pest control agent or pharmaceutical agent and being less volatile than the volatile solvent.

2. An impregnation method as claimed in claim 1, further characterized in that the volatile solvent/swelling agent is lipophilic, and the impregnation material is fatsoluble.

3. An impregnation method as claimed in claim 1 or 2, further characterized in that the swelling agents is carbon dioxide or nitrous oxide.

4. An impregnation method as claimed in claim 1, 2 or 3, further characterized in that the impregnation material is musk, rose oil, honeysuckle oil, pine oil, and jasmine or oak musk.

5. An impregnation method as claimed in claim 1, 2 or 3, further characterized in that the impregnation material is a pheromone or a pyrethrin.

6. An impregnation method as claimed in claim 1, 2 or 3, further characterized in that the impregnation material is scopolamine.

## Patentansprüche

1. Verfahren zum Imprägnieren eines Feststoffes mit einem Imprägnierungsmaterial, bei dem der Feststoff mit einem flüchtigen Lösungmittel, in dem das Imprägnierungsmaterial gelöst ist, in Berührung gebracht und danach der Druck vermindert wird, dadurch gekennzeichnet, dass der Feststoff ein thermoplastisches Polymer ist und das Lösungsmittel auch als Quellmittel für das Polymer dient, dass das Polymer mit dem Lösungsmittel, welches das Imprägnierungsmaterial gelöst enthält, bei den überkritischen Bedingungen des Lösungsmittels für Temperatur und Druck oder in deren Nähe in Berührung gebracht und seine Quellung bewirkt wird, und dass der Druck erniedrigt wird, um ein Ausdiffundieren des Lösungsmittels aus dem ququollenen Polymer zu bewirken, wobei als Imprägnierungsmaterial ein Duftstoff, ein Schädlingsbekämpfungsmittel oder ein pharmazeutisches Mittel verwendet wird, dessen Flüchtigkeit geringer ist als diejenige des flüchtigen Lösungsmittels.

2. Imprägnierungsverfahren nach Anspruch 1, dadurch gekennzeichnet, dass das flüchtige Lösungs- bzw. Quellmittel lipophil ist und das Imprägnierungsmaterial fettlöslich ist.

3. Imprägnierungsverfahren nach Anspruch 1 oder 2, dadurch gekennzeichnet, dass das Quellmittel Kohlendioxyd oder Distickstoffoxyd ist.

4. Imprägnierungsverfahren nach Anspruch 1, 2 oder 3, dadurch gekennzeichnet, dass das Imprägnierungsmaterial Moschus, Rosenöl, Geissblattöl, Fichtenöl und Jasmin- oder Moschuskörneröl ist.

5. Imprägnierungsverfahren nach Anspruch 1, 2 oder 3, dadurch gekennzeichnet, dass das Imprägnierungsmaterial ein Pheromon oder ein Pyrethrin ist.

6. Imprägnierungsverfahren nach Anspruch 1, 2 oder 3, dadurch gekennzeichnet, dass das Imprägnierungsmaterial Scopolamin ist.

## Revendications

1. Procédé d'imprégnation d'un solide par un matériau d'imprégnation par mise en contact du solide avec un solvant volatil dans lequel le matériau odorant est dissous et ensuite la pression réduite, caractérisé en ce que le solide est un polymère thermoplastique et le solvant également un agent gonflant pour le polymère, le polymère est mis en contact avec et gonflé par le solvant contenant le matériau d'imprégnation en solution dans ou proche des conditions surcritiques de température et de pression du solvant, et la pression est réduite pour diffuser le solvant hors du polymère thermoplastique gonflé, le matériau d'imprégnation étant une fragrance ou un agent pesticide ou un agent pharmaceutique et étant moins volatil que le solvant volatil.

2. Procédé d'imprégnation selon la revendication 1, caractérisé en ce que l'agent volatil solvant/gonflant est lipophile, et le matériau d'imprégnation soluble dans la graisse.

3. Procédé d'imprégnation selon la revendication 1 ou 2, caractérisé en ce que l'agent gonflant est du dioxyde de carbone ou de l'oxyde azoté.

4. Procédé d'imprégnation selon la revendication 1, 2 ou 3, caractérisé en ce que le matériau d'imprégnation est du musc, de l'essence de roses, de l'essence de chèvrefeuille, de l'essence de pin, et du musc de jasmin ou de chêne.

5. Procédé d'imprégnation selon la revendication 1, 2 ou 3, caractérisé en ce que le matériau d'imprégnation est une phéromone ou une pyréthrine.

6. Procédé d'imprégnation selon la revendication 1, 2 ou 3, caractérisé en ce que le matériau d'imprégnation est la scopolamine.